# EUROPÄISCHE PATENTSCHRIFT

(11) **EP 3 623 006 B1**
(45) Veröffentlichungstag und Bekanntmachung des Hinweises auf die Patenterteilung: **27.07.2022**
(21) Anmeldenummer: 18194057.8
(22) Anmeldetag: 12.09.2018
(51) Int. Cl.: A61N 1/375, A61N 1/378

(54) **FIXIERUNG VON KOMPONENTEN IM INNENRAUM EINES IMPLANTIERBAREN MEDIZINISCHEN GERÄTS MITTELS EINES MONTAGERAHMENS**
FIXATION OF COMPONENTS IN THE INTERIOR OF AN IMPLANTABLE MEDICAL DEVICE WITH A MOUNTING FRAME
FIXATION DE COMPOSANTS DANS L'ESPACE INTÉRIEUR D'UN APPAREIL MÉDICAL IMPLANTABLE AU MOYEN D'UN CADRE DE MONTAGE

(43) Veröffentlichungstag der Anmeldung: 18.03.2020
(73) Patentinhaber: BIOTRONIK SE & Co. KG, 12359 Berlin (DE)
(72) Erfinder: HENSCHEL, Martin, 13125 Berlin (DE); NEUMANN, Wiebke, 12209 Berlin (DE)
(74) Vertreter: Biotronik Corporate Services SE

(56) Entgegenhaltungen:
- WO-A1-2015/106109
- US-A- 5 814 090
- US-A- 5 873 899
- US-A1- 2004 082 977
- US-A1- 2017 127 543

## Beschreibung

Die Erfindung betrifft ein implantierbares medizinisches Gerät, insbesondere einen Kardioverter- Defibrillator.

Implantierbare medizinisches Geräte, insbesondere Implantate zur Stimulation eines Organs und/oder zur Aufnahme eines physiologischen Signals (z.B. implantierbare Herzschrittmacher, Kardioverter-Defibrillatoren (ICD), Neurostimulatoren etc.), weisen in der Regel ein Gerätegehäuse auf, in dem zumindest eine elektrische Komponente angeordnet ist.

Mithilfe eines derartigen ICDs kann der Herzrhythmus einer Person überwacht werden, wobei bei detektierten Unregelmäßigkeiten, insbesondere bei Kammerflimmern, Kammerflattern oder einer Tachykardie, ein elektrischer Impuls durch den ICD an das Herz abgegeben werden kann, durch den ein normaler Herzrhythmus wieder hergestellt werden kann.

Für eine uneingeschränkte Funktion solcher implantierbaren medizinischen Geräte muss insbesondere eine Verbindung zwischen einer Leiterplatte/Schaltkreis und einer elektrischen Komponente (z.B. Batterie oder Kondensator) des Gerätes bestehen, die auch bei einer Bewegung des implantierbaren medizinischen Gerätes intakt bleibt. Um dies zu erreichen, kann die elektrische Komponente im implantierbaren medizinischen Gerät fixiert werden. Der Begriff "Fixierung" meint dabei, dass eine Bewegung der Komponente bezüglich des Gerätegehäuses verhindert wird. Bei einer "vollständigen Fixierung" wird eine Bewegung einer Komponente in alle Raumrichtungen verhindert. Bei einer "unvollständigen Fixierung" kann sich eine Komponente zumindest in eine Raumrichtung bewegen. Ohne eine Fixierung oder bei einer unvollständigen Fixierung der Komponente kann sich die Komponente innerhalb des Gehäuses des implantierbaren medizinischen Gerätes bewegen, so dass z.B. die besagte Verbindung zwischen der Komponente und der Leiterplatte/Schaltkreis unterbrochen bzw. gestört werden kann. Dies kann einen Funktionsausfall zur Folge haben, wodurch es möglicherweise zu einem unerwünschten Therapieausfall kommen kann.

Auch ist für den Herstellungsprozess eines implantierbaren medizinischen Geräts ist eine Fixierung von wesentlichen Komponenten innerhalb des Montagerahmens. Unter einem Montagerahmen wird im Allgemeinen ein Element verstanden, das dazu ausgelegt ist, Komponenten eines Gerätes zu fixieren. Ein Montagerahmen wird verwendet, um Herstellungsschritte zu vereinfachen und einen sicheren Transport von montierten Produkten und Zwischenprodukten zu gewährleisten.

Zur Reduktion einer Bewegung einer Komponente im Inneren des Gerätes ist im Stand der Technik ein Montagerahmen bekannt, in den eine Komponente positioniert werden kann. Ein derartiger Montagerahmen kann sogenannte Rippen aufweisen, mit deren Hilfe die Komponente in einer ersten Ebene fixiert werden kann, d.h., dass die Bewegung der Komponente parallel zur ersten Ebene reduziert oder verhindert werden kann. Bei einer Positionierung der Komponente in einem solchen Montagerahmen kann sich die Komponente jedoch weiterhin senkrecht zur ersten Ebene bewegen. Eine vollständige Fixierung ist daher durch einen derartigen Montagerahmen nicht erreicht.

Für eine Fixierung der Komponenten senkrecht zur ersten Ebene sind im Stand der Technik unterschiedliche Lösungen offenbart.

Eine Lösung sieht eine zusätzliche Fixierung einer Komponente durch Kleben vor. Allerdings kann ein dabei verwendetes Klebematerial Alterungserscheinungen unterliegen, so dass die Fixierung mit zunehmendem Alter des implantierbaren medizinischen Gerätes an Wirksamkeit verlieren kann. Weiterhin würde bei einer derartigen Fixierung die Herstellungszeit des implantierbaren medizinischen Gerätes verlängert, da das Klebematerial aushärten muss, bevor ein weiterer Herstellungsschritt erfolgen kann. Weiterhin könnten Bestandteile des Klebematerials für Veränderungen anderer Komponenten des implantierbaren medizinischen Gerätes, insbesondere der Leiterplatte, sorgen. Insbesondere könnte es zu einer Korrosion kommen, durch die die Funktion der Leiterplatte und damit die Funktion des implantierbaren medizinischen Gerätes reduziert werden könnte.

Nach dem Stand der Technik ist alternativ eine Lösung bekannt, die ein Pressen des implantierbaren medizinischen Gerätes umfasst. Dabei besteht jedoch unter Umständen das Risiko einer Beschädigung der Komponenten bzw. das Risiko, dass das Gehäuse nicht vollständig geschlossen wird.

Eine andere Lösung weist ein zusätzliches Bauteil auf, wobei das zusätzliche Bauteil der Fixierung durch Ausgleich einer Toleranz dienen kann. Ein zusätzliches Bauteil kann beispielsweise eine zusätzliche Kunststofffolie oder ein Element aus Schaumstoff sein. Allerdings können durch zusätzliche Bauteile die Kosten der Herstellung des implantierbaren medizinischen Gerätes in unerwünschter Weise erhöht werden. Schließlich wird in der WO 2015/106109 ein erster Rahmen offenbart, der Rückhalteelemente aufweist, durch die ein zweiter Rahmen, der im Inneren des ersten Rahmens positioniert ist, stabilisiert werden kann. US 2017/127543, US 5 814 090, US 2004/082977 und US 5 873 899 zeigen weitere Ausführungen von Montagerahmen.

Hiervon ausgehend liegt der vorliegenden Erfindung die Aufgabe zugrunde, ein implantierbares medizinisches Gerät bereitzustellen, das eine elektrische Komponente aufweist, die auf einfache Weise sicher im Innenraum des Gerätegehäuses fixierbar ist. Weiterhin soll das implantierbare medizinische Gerät insbesondere so gestaltet sein, dass die Komponente auf einfache Weise im Innenraum des Gerätegehäuses anordbar ist.

Diese Aufgabe wird durch ein implantierbares medizinisches Gerät mit den Merkmalen des Anspruchs 1 gelöst. Vorteilhafte Ausführungsformen sind in den Unteransprüchen angegeben und werden nachfolgend beschrieben.

Gemäß Anspruch 1 wird ein implantierbares medizinisches Gerät offenbart, mit: einem Gerätegehäuse, das eine erste und eine zweite Gerätegehäuseschale aufweist, wobei das Gerätegehäuse einen Innenraum umgibt. Ferner weist das implantierbare medizinische Gerät eine elektrische Komponente des Gerätes auf, die im Innenraum angeordnet ist. Zudem weist das implantierbare medizinische Gerät einen Montagerahmen auf, der im Innenraum angeordnet ist und die elektrische Komponente umgibt. Dabei weist der Montagerahmen mindestens eine Klemmstruktur auf, wobei die mindestens eine Klemmstruktur dazu ausgebildet ist, eine Kraft auf die elektrische Komponente auszuüben, um die elektrische Komponente im Innenraum des Gerätegehäuses zu fixieren, wobei die mindestens eine Klemmstruktur als eine Wippe ausgebildet ist, wobei die Wippe zumindest einen ersten Arm und einen zweiten Arm aufweist, wobei der erste Arm und der zweite Arm in unterschiedliche Richtungen von einem durch den Montagerahmen gebildeten Steg abstehen.

Ein implantierbares medizinisches Gerät wird im Folgenden auch kurz als Implantat bezeichnet.

In einer Ausführungsform sind das Gerätegehäuse und der Montagerahmen dazu ausgebildet eine Leiterplatte und mindestens eine, insbesondere zwei, elektrische Komponenten zu umgeben bzw. aufzunehmen.

Der Montagerahmen kann insbesondere einstückig ausgebildet sein. Der Montagerahmen kann dabei ein Material aufweisen oder aus einem Material gebildet sein, das Toleranzen der Bauteile des Implantats ausgleichen kann. Das Material kann ein elastisches Material sein. Insbesondere kann das Material eines der folgenden Materiale sein: Polybutylenterephthalat (PBTP, PBT), Liquid Crystal Polymer (LCP), Polyurethan (PU), Acrylnitril-Butadien-Styrol (ABS), Polysolfon (PSU), Polyetheretherketon (PEEK), Polyoxymethylen (POM), etc.

Die erste und/oder die zweite Gerätegehäuseschale können beispielsweise eines der folgenden Materiale aufweisen oder durch eines der folgenden Materialien gebildet sein: Titan, oder eine Titanlegierung (biokompatibel).

Der Montagerahmen kann des Weiteren in der ersten und/oder der zweiten Gerätegehäuseschale angeordnet sein.

In einer Ausführungsform der Erfindung ist die mindestens eine Klemmstruktur dazu ausgebildet, mindestens einen ersten und einen zweiten Zustand einzunehmen, wobei im ersten Zustand keine Kraft durch die mindestens eine Klemmstruktur auf die elektrische Komponente ausgeübt wird, und wobei im zweitem Zustand eine Kraft durch die mindestens eine Klemmstruktur auf die elektrische Komponente ausgeübt wird, um die Komponente im Innenraum des Gerätegehäuses zu fixieren.

Ferner kann die mindestens eine Klemmstruktur dazu ausgebildet und derart positioniert sein, dass durch die Klemmstruktur im ersten Zustand die Anordnung bzw. Montage der elektrischen Komponente im Montagerahmen nicht gestört und/oder verhindert wird.

Die Anordnung bzw. Montage kann insbesondere entlang einer Montagerichtung durchgeführt werden, d.h. die elektrische Komponente kann entlang einer Montagerichtung in dem Montagerahmen angeordnet werden bzw. in diesen eingesetzt werden.

Gemäß einer weiteren Ausführungsform der Erfindung ist die mindestens eine Klemmstruktur dazu ausgebildet, vom ersten Zustand in den zweiten Zustand überführt zu werden, wenn die elektrische Komponente in dem Montagerahmen angeordnet wird.

Das bedeutet, dass für die Überführung der mindestens einen Klemmstruktur vom ersten Zustand in den zweiten Zustand kein zusätzlicher Herstellungsschritt bei der Herstellung des Implantates benötigt wird, da durch die Anordnung der elektrischen Komponente in dem Montagerahmen die elektrische Komponente vom ersten in den zweiten Zustand überführt und die elektrischen Komponente im Innenraum fixiert werden kann.

In einer alternativen Ausführungsform ist die mindestens eine Klemmstruktur dazu ausgebildet vom ersten Zustand in den zweiten Zustand überführt zu werden, wenn das Gerätegehäuse durch Zusammensetzen der ersten und der zweiten Gerätegehäuseschale geschlossen wird.

Durch das Schließen des Gerätegehäuses kann die elektrische Komponente vom ersten in den zweiten Zustand überführt und die elektrischen Komponente im Innenraum fixiert werden, so dass die Überführung vom ersten Zustand in den zweiten Zustand ein zusätzliches Resultat eines Herstellungsschrittes des Implantates ist, der in jedem Falle durchgeführt werden muss. Das bedeutet, dass für die Überführung vom ersten Zustand in den zweiten Zustand, d.h. für das Fixieren der elektrischen Komponente, kein zusätzlicher Herstellungs- bzw. Verfahrensschritt durchgeführt werden muss.

Gemäß einer weiteren Ausführungsform der Erfindung ist vorgesehen, dass die erste Gerätegehäuseschale einen Boden sowie vorzugsweise eine davon abgehende umlaufende Wandung aufweist. Weiterhin ist gemäß einer Ausführungsform der Erfindung vorgesehen, dass die zweite Gerätegehäuseschale einen Boden sowie vorzugsweise eine davon abgehende umlaufende Wandung aufweist.

Der Boden der ersten Gerätegehäuseschale kann sich dabei in bzw. entlang einer ersten Ebene erstrecken. In gleicher Weise kann sich der Boden der zweiten Gerätegehäuseschale kann in bzw. entlang einer zweiten Ebene erstrecken.

Bei einem geschlossenen Gerätegehäuse können sich somit die erste Ebene und die zweite Ebene parallel zueinander erstrecken, d.h., der Boden der ersten Gerätegehäuseschale kann parallel zum Boden der zweiten Gerätegehäuseschale verlaufen. Die jeweilige Gerätegehäuseschale kann insbesondere einstückig ausgebildet sein.

Denkbar sind ebenfalls abgerundete Formen eines Gerätegehäuses, deren Gehäuseschalen keine planen Ebenen aufweisen.

Der Boden der ersten und/oder der zweiten Gerätegehäuseschale und der Montagerahmen können so konfiguriert sein, dass der Montagerahmen im Innenraum auf dem Boden der ersten und/oder der zweiten Gerätegehäuseschale anordenbar ist. Insbesondere kann der Montagerahmen so in der ersten und/oder der zweiten Gerätegehäuseschale anordenbar bzw. angeordnet sein, dass eine elektrische Komponente entlang einer Montagerichtung, die senkrecht zu dem besagten Boden verläuft, in dem Montagerahmen angeordnet werden kann.

Die mindestens eine Klemmstruktur kann dazu ausgebildet sein, eine Kraft auf die elektrische Komponente auszuüben, die zumindest eine Vektorkomponente aufweist, die sich senkrecht zum Boden der ersten oder zweiten Gerätegehäuseschale erstreckt. Das bedeutet, dass die elektrische Komponente mithilfe der mindestens einen Klemmstruktur insbesondere senkrecht zu dem besagten Boden des Gerätegehäuses fixiert werden kann. Weiterhin ist gemäß einer Ausführungsform des implantierbaren medizinischen Gerätes

vorgesehen, dass mindestens eine weitere Klemmstruktur vorgesehen ist, wobei die mindestens eine weitere Klemmstruktur als ein Vorsprung des Montagerahmens ausgebildet ist, wobei sich der Vorsprung des Montagerahmens im ersten Zustand der mindestens einen Klemmstruktur senkrecht zum Boden der ersten Gerätegehäuseschale oder senkrecht zum Boden der zweiten Gerätegehäuseschale erstreckt.

Der Vorsprung kann sich dabei im ersten Zustand entlang der Montagerichtung erstrecken, die senkrecht zum Boden der ersten oder der zweiten Gerätegehäuseschale verläuft (siehe oben). Die elektrische Komponente kann daher leicht entlang der Montagerichtung in dem Montagerahmen angeordnet werden, ohne dass die mindestens eine Klemmstruktur bzw. der mindestens eine Vorsprung die Montage beeinträchtigt und/oder verhindert.

Weiterhin ist gemäß einer Ausführungsform der Erfindung vorgesehen, dass der Vorsprung des Montagerahmens ein freies Ende aufweist, wobei sich der Vorsprung des Montagerahmens zu diesem Ende hin verjüngt.

Gemäß einer Ausführungsform der Erfindung ist weiterhin vorgesehen, dass zumindest ein Bereich der mindestens einen Klemmstruktur (z.B. des Vorsprungs oder der Wippe, siehe unten) deformierbar ausgebildet ist. Durch eine Deformation dieses Bereiches der jeweiligen Klemmstruktur ist die betreffende Klemmstruktur von ihrem ersten Zustand in ihren zweiten Zustand überführbar.

In einer Ausführungsform der Erfindung ist die mindestens eine Klemmstruktur bzw. der mindestens eine Vorsprung durch das Schließen des Gerätegehäuses, d.h., durch das bestimmungsgemäße Verbinden der beiden Gerätegehäuseschalen, deformierbar, so dass die mindestens eine Klemmstruktur (z.B. Vorsprung) vom ersten Zustand in den zweiten Zustand überführt wird, in dem die mindestens eine Klemmstruktur die Komponente im Innenraum des Gerätegehäuses fixiert.

Für den Fall, dass der Montagerahmen und die elektrische Komponente bei der Montage in der ersten Gerätegehäuseschale angeordnet werden, kann der mindestens eine Vorsprung des Montagerahmens senkrecht zum Boden der ersten Gerätegehäuseschale über deren Wandung hinausragen, wobei die zweite Gerätegehäuseschale insbesondere dazu ausgebildet sein kann, beim Verbinden mit der ersten Gerätegehäuseschale gegen den mindestens einen Vorsprung zu drücken, so dass dieser vom ersten in den zweiten Zustand überführt wird. Alternativ hierzu kann der Montagerahmen bzw. die Komponente zunächst auch in der zweiten Gerätegehäuseschale angeordnet werden, wobei das Fixieren dann durch Verbinden der ersten Gerätegehäuseschale mit der zweiten Gerätegehäuseschale erfolgt, in der der Rahmen und die Komponente ruhen.

Der Umstand, dass zumindest ein Bereich der jeweiligen Klemmstruktur deformierbar ist, um die Klemmstruktur vom ersten in den zweiten Zustand zu überführen, kann insbesondere auch bedeuten, dass ein anderer Bereich der betreffenden Klemmstruktur vorliegen kann, der bei der Überführung vom ersten in den zweiten Zustand nicht deformiert wird, also mit anderen Wort bei der Überführung seine Form behält.

So kann z.B. gemäß einer weiteren Ausführungsform der Erfindung vorgesehen sein, dass die mindestens eine Klemmstruktur im zweiten Zustand in Richtung auf den Boden der ersten oder zweiten Gerätegehäuseschale geneigt sein kann und dabei gegen die elektrische Komponente drückt, um die besagte Kraft auf die elektrische Komponente auszuüben und diese im Innenraum des Gerätegehäuses zu fixieren. Die Klemmstruktur kann z.B. im zweiten Zustand in Richtung des Bodens der ersten Gerätegehäuseschale geneigt sein, wenn der Montagerahmen bei der Montage zunächst in der ersten Gerätegehäuseschale angeordnet ist. Für den Fall, dass der Montagerahmen bei der Montage zunächst in der zweiten Gerätegehäuseschale angeordnet wird, kann die Klemmstruktur im zweiten Zustand in Richtung des Bodens der zweiten Gerätegehäuseschale geneigt sein Insbesondere kann die Klemmstruktur wiederum im zweiten Zustand mit einer Kraft gegen die Komponente drücken, die eine Vektorkomponente aufweist, die senkrecht zum Boden der ersten oder der zweiten Gerätegehäuseschale verläuft, so dass die Klemmstruktur die elektrische Komponente senkrecht zum betreffenden Boden des Gerätegehäuses fixieren kann.

Dass die Klemmstruktur im zweiten Zustand in Richtung des Bodens der ersten oder der zweiten Gerätegehäuseschale geneigt sein kann bedeutet insbesondere, dass sich der Vorsprung unter einem spitzen Winkel zum Boden der ersten oder zweiten Gerätegehäuseschale erstreckt. Entsprechend kann ein Abstand zwischen dem Ende des Vorsprungs und dem Boden der ersten oder der zweiten Gerätegehäuseschale im zweiten Zustand geringer sein als im ersten Zustand.

der Erfindung ist vorgesehen, dass die mindestens eine Klemmstruktur als eine Wippe ausgebildet ist. Dabei weist die Wippe zumindest einen ersten und einen zweiten Arm auf, wobei der erste Arm und der zweite Arm in unterschiedliche Richtungen von einem durch den Montagerahmen gebildeten Steg abstehen.

Gemäß einer Ausführungsform der Erfindung können der Montagerahmen und die Wippe einstückig ausgebildet sein, d.h., aus einem Stück gefertigt sein (z.B. durch einen Formgebungsprozess wie z.B. Spritzgießen). Es ist auch denkbar, den Montagerahmen und die Wippe getrennt voneinander zu fertigen und anschließend miteinander zu verbinden. Gemäß einer Ausführungsform der Erfindung kann weiterhin vorgesehen sein, dass der erste und der zweite Arm in einer Ebene von dem Steg abstehen, die sich senkrecht zu einer Längserstreckungsrichtung des Steges und insbesondere senkrecht zum Boden der ersten oder zweiten Gerätegehäuseschale erstreckt. Die Längserstreckungsrichtung bzw. Längsachse des Steges kann entlang einer Umfangsrichtung des Montagerahmens verlaufen entlang der Montagerahmen umläuft.

Gemäß einer weiteren Ausführungsform der Erfindung ist vorgesehen, dass sich der erste Arm der Wippe im ersten Zustand der Wippe geneigt zum Boden der ersten oder der zweiten Gerätegehäuseschale erstreckt und/oder dass sich der zweite Arm der Wippe im ersten Zustand der Wippe senkrecht zum Boden der ersten oder der zweiten Gerätegehäuseschale erstreckt.

Der erste Arm und der zweite Arm der Wipp/Klemmstruktur können winklig zueinander angeordnet sein. Ein entsprechend zwischen den beiden Armen gebildeter Winkel kann ein stumpfer Winkel zwischen 90° und 175° sein, der insbesondere zwischen 100° und 170°, insbesondere zwischen 110° und 160° liegt. Insbesondere kann der Winkel 150° betragen.

Im ersten Zustand der Wippe kann sich z.B. der zweite Arm senkrecht oder in einem Winkel größer als 90° zum Boden der ersten Gerätegehäuseschale erstrecken, so dass die elektrische Komponente entlang der Montagerichtung montiert werden kann, d.h., in den in der ersten Gerätegehäuseschale angeordneten Montagerahmen angeordnet werden kann, ohne mit dem zweiten Arm zu kollidieren. Das heißt, dass der zweite Arm eine Anordnung der elektrischen Komponente in den Montagerahmen bzw. in den Innenraum des Gerätegehäuses weder erschwert noch verhindert.

Weiterhin ist gemäß einer Ausführungsform der Erfindung vorgesehen, dass der zweite Arm der Wippe länger ist als der erste Arm der Wippe.

Weiterhin ist gemäß einer Ausführungsform der Erfindung vorgesehen, dass die mindestens eine im ersten Zustand befindliche Klemmstruktur bzw. Wippe durch eine Kraftausübung auf den ersten Arm in den zweiten Zustand kippbar ist. Die Klemmstruktur bzw. Wippe kann um eine Kippachse, die entlang der Längserstreckungsrichtung des Steges verlaufen kann, gekippt werden, wobei der Steg beim Kippen der Wippe/Klemmstruktur deformiert wird. Insbesondere ist vorgesehen, dass die Wippe durch Ausüben einer Kraft auf den ersten Arm vom ersten in den zweiten Zustand kippbar ist, wobei jene Kraft eine Vektorkomponente aufweist, die senkrecht zum Boden der ersten oder der zweiten Gerätegehäuseschale verläuft. Insbesondere kann der Montagerahmen so ausgebildet sein, dass die Klemmstruktur bzw. Wippe vom ersten in den zweiten Zustand kippt, wenn die elektrische Komponente im Montagerahmen angeordnet wird. Das heißt mit anderen Worten, dass über die elektrische Komponente jene Kraft auf den ersten Arm der Wippe ausgeübt wird, wenn die Komponente in den z.B. in der ersten Gerätegehäuseschale ruhenden Montagerahmen eingeführt wird und dabei in der Montagerichtung gegen den ersten Arm der Wippe drückt, so dass diese in den fixierenden zweiten Zustand gekippt wird, in dem der zweite Arm der Wippe gehen die Komponente drückt und diese im Innenraum bzw. im Montagerahmen fixiert. Im zweiten Zustand kann sich dabei der zweite Arm der Wippe geneigt zum Boden der ersten Gerätegehäuseschale erstrecken. Der erste Arm kann sich im zweiten Zustand senkrecht oder geneigt zum Boden der ersten Gerätegehäuseschale erstrecken.

Insbesondere ist gemäß einer Ausführungsform der Erfindung vorgesehen, dass der zweite Arm der Wippe im zweiten Zustand der Wippe gegen die elektrische Komponente mit einer Kraft drückt, die eine Vektorkomponente senkrecht zum Boden der ersten Gerätegehäuseschale aufweist.

Weiterhin ist gemäß einer Ausführungsform der Erfindung vorgesehen, dass der Montagerahmen einen ersten und einen gegenüberliegenden zweiten Rand aufweist. Dabei kann der Steg zwischen einer Aussparung des Montagerahmens am ersten Rand und einer gegenüberliegenden Aussparung des Montagerahmens am zweiten Rand verlaufen. Der Steg kann also mit anderen Worten als Verengung des Montagerahmens ausgebildet sein.

Grundsätzlich kann gemäß einer weiteren Ausführungsform der Erfindung vorgesehen sein, dass der Montagerahmen eine weitere Klemmstruktur oder eine Vielzahl an Klemmstrukturen aufweist. Bei der jeweiligen Klemmstruktur kann es sich um einen Vorsprung des Montagerahmens handeln, der wiederum gemäß den hierin beschriebenen Ausführungsformen ausgebildet sein. Weiterhin kann es sich bei der jeweiligen Klemmstruktur um eine hierin beschriebene Wippe handeln. Eine Kombination von Vorsprüngen des Montagerahmens und Wippen ist ebenfalls denkbar.

Einzelne Klemmstrukturen der Vielzahl an Klemmstrukturen können an unterschiedlichen Positionen entlang der Umfangsrichtung des Montagerahmens positioniert sein. Zwischen einer ersten Klemmstruktur und einer zweiten Klemmstruktur kann ein Abstand bestehen, der sich von einem Abstand zwischen der zweiten Klemmstruktur und einer dritten Klemmstruktur unterscheidet. In einer alternativen Ausführungsform kann der Abstand zwischen je zwei in Umfangsrichtung benachbarten Klemmstrukturen äquidistant sein.

Eine Fixierung der elektrischen Komponente durch eine Vielzahl an Klemmstrukturen (z.B. Vorsprünge und/oder Wippen) kann insoweit von Vorteil sein, da hier die fixierende Kraft auf eine Vielzahl an Klemmstrukturen bzw. auf eine Vielzahl an Kontaktstellen zur elektrischen Komponente verteilt ist. Eine fehlerhafte Funktion einer individuellen Klemmstruktur kann entsprechend durch eine oder mehrere andere Klemmstrukturen kompensiert werden.

Ferner können durch mehrere Klemmstrukturen auf einfache Weise mehrere elektrische Komponenten des Implantats im Innenraum des Gerätegehäuses bzw. im Montagerahmen fixiert werden. In einer Ausführungsform kann jeweils zumindest eine Klemmstruktur eine Kraft auf jeweils eine zugeordnete elektrische Komponente ausüben.

Weiterhin ist der Erfindung vorgesehen, dass die mindestens eine elektrische Komponente des Implantats eine Batterie, ein Kondensator oder eine Kondensatoranordnung ist (z.B. ein Kondensatorstapel). Die Batterie kann insbesondere Energie für den Betrieb des medizinischen Implantates liefern. Der Kondensator kann insbesondere der Bereitstellung von elektrischen Impulsen zur Herstellung des normalen Herzrhythmus (Defibrillationsenergie) dienen.

Weitere Merkmale und Vorteile der Erfindung werden nachfolgend anhand der Figurenbeschreibung von Ausführungsbeispielen erläutert. Es zeigen:
- Fig. 1: einen Querschnitt einer ersten Gerätegehäuseschale mit darin angeordneten elektrischen Komponenten,
- Fig. 2: einen Querschnitt einer ersten Gerätegehäuseschale mit darin angeordnetem Montagerahmen und Montagerichtung,
- Fig. 3: eine perspektivische Ansicht eines Montagerahmens,
- Fig. 4: einen Querschnitt einer Klemmstruktur in Form einer Wippe,
- Fig. 5A: eine perspektivische Detailansicht eines Montagerahmens mit einer Wippe im ersten Zustand,
- Fig. 5B: eine perspektivische Detailansicht eines Montagerahmens mit einer Wippe im zweiten Zustand,
- Fig. 6A: eine Detailansicht eines Montagerahmens mit einem Vorsprung im ersten Zustand, und
- Fig. 6B: eine Detailansicht eines Montagerahmens mit einem Vorsprung im zweiten Zustand.

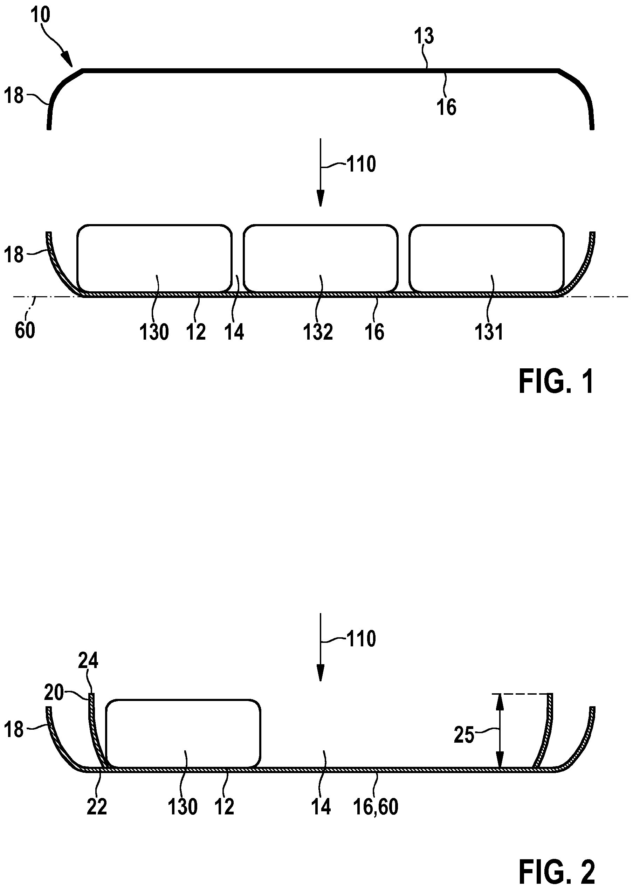

Figur 1 zeigt einen schematischen Querschnitt durch eine erste Gerätegehäuseschale 12 eines Gerätegehäuses eines erfindungsgemäßen Implantats 1. Die erste Gerätegehäuseschale 12 kann einen Boden 16 und eine Wandung 18 aufweisen. Der Boden 16 der ersten Gerätegehäuseschale 12 kann sich in einer ersten Ebene 60 erstrecken. Die Wandung 18 kann insbesondere eine umlaufende Wandung 18 sein.

In der ersten Gerätegehäuseschale 12 ist zumindest eine elektrische Komponente 130, 131, 132 angeordnet. Figur 1 zeigt diesbezüglich schematisch als Beispiel eine Leiterplatte 132 bzw. einen Schaltkreis 132 sowie weitere elektrische Komponenten 130, 131, bei denen es sich z.B. um eine Batterie 130 und einen Kondensator 131 handeln kann. Der Kondensator 131 kann in Form eines Kondensatorstapels 131 vorliegen. Die Leiterplatte 132 kann zwischen den beiden weiteren elektrischen Komponenten 130, 131 positioniert sein.

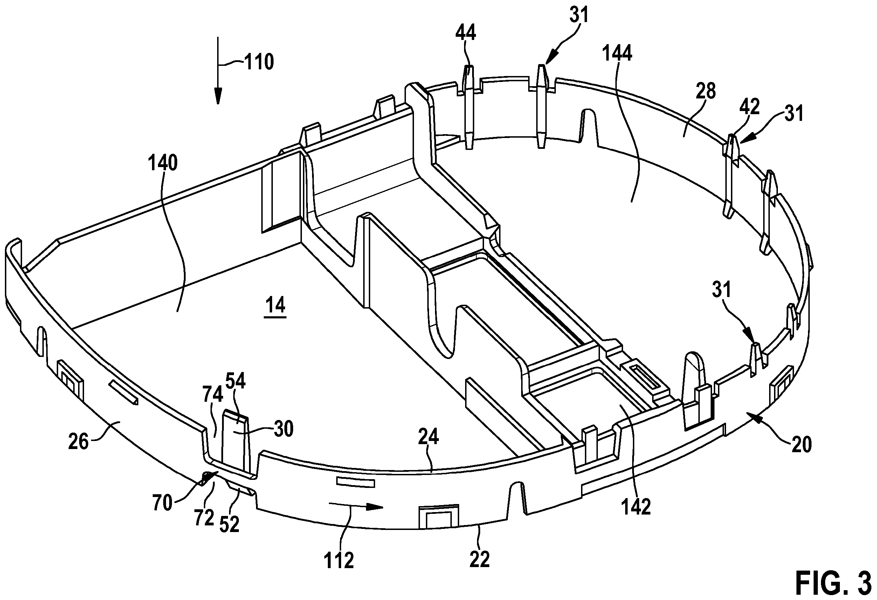

Die elektrischen Komponenten 130, 131, 132 können entlang einer Montagerichtung 110, illustriert durch den Pfeil in Fig. 1, in die erste Gerätegehäuseschale 12 eingebracht werden. Die Montagerichtung 110 verläuft vorzugsweise senkrecht zur ersten Ebene 60 bzw. zum Boden 16 der Gerätegehäuseschale 12. Hiernach kann zur Ausbildung des Gerätegehäuses 10 eine zweite Gerätegehäuseschale 13 mit der ersten (hier unteren) Gerätegehäuseschale 12 verbunden werden. Die Komponenten 130, 131, 132 können mittels eines in der Figur 2 gezeigten Montagerahmens 20 im Innenraum 14 des Gerätegehäuse 10 fixiert werden. Ein solcher Montagerahmen 20 ist schematisch in der Figur 2 dargestellt. Figur 3 zeigt eine perspektivische Ansicht einer Ausführungsform eines solchen Montagerahmens 20, der hier beispielsweise zum Positionieren einer Batterie 130, eines Kondensators 131 sowie einer Leiterplatte 132 dient.

Gemäß Figur 2 kann der Montagerahmen 20 im Innenraum 14 bzw. in der ersten Gerätegehäuseschale 12 positioniert sein, so dass der Montagerahmen 20 zumindest einen Teilbereich des Innenraumes 14 umgeben kann. Der Montagerahmen 20 kann einen ersten Rand 22 aufweisen und mit diesem auf dem Boden 16 der Schale 12 aufliegen. Der Montagerahmen 20 weist insbesondere einen gegenüberliegenden zweiten Rand 24 auf, wobei eine Distanz zwischen den beiden Rändern 22, 24 einer Höhe 25 des Montagerahmens 20 entspricht. Die mindestens eine elektrischen Komponente 130, 131, 132 (in der Figur 2 ist exemplarisch nur die Batterie 130 dargestellt) wird vorzugsweise in Montagerichtung 110, d.h., senkrecht zur ersten Ebene 60 bzw. zum Boden 60 in den Montagerahmen 20 eingesetzt.

In Figur 3 ist eine perspektivische Ansicht eines erfindungsgemäßen Montagerahmens 20 gezeigt. Der Montagerahmen 20 kann zumindest einen Teilbereich des Innenraumes 14 des Gerätegehäuses 10 umgeben und kann dabei so gestaltet sein, dass er den Innenraum 14 in einen ersten Bereich 140, einen zweiten Bereich 142 und einen dritten Bereich 144 unterteilt. Der zweite Bereich 142 kann dazu ausgebildet sein, eine Leiterplatte zu umgeben. Der erste und der dritte Bereich 140, 144 können zur Aufnahme je einer elektrischen Komponente 130, 131 ausgebildet sein (z.B. in Form einer Batterie 130 und eines Kondensators 131).

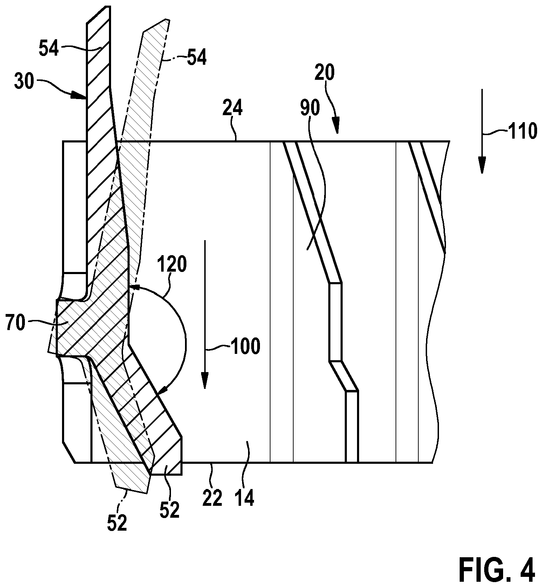

Es ist vorgesehen, dass der Montagerahmen 20 zumindest eine oder mehrere Klemmstrukturen 30, 31 aufweist. Der Montagerahmen 20 und die besagten Klemmstrukturen 30, 31 sind des Weiteren in den Figuren 4, 5A, 5B, 6A und 6B detailliert dargestellt.

Die mindestens eine Klemmstruktur 30, 31 kann z.B. in Form einer Wippe 30 ausgebildet sein. In einem alternativen Beispiel kann die mindestens eine Klemmstruktur 31 durch einen Vorsprung 31 des Montagerahmens 20 ausgebildet sein.

Insbesondere kann der Montagerahmen 20 gemäß Figur 3 eine Vielzahl an Klemmstrukturen 30, 31 aufweisen, und zwar insbesondere zumindest eine Klemmstruktur 30 in Form einer Wippe 30 sowie mehrere Klemmstrukturen 31 in Form je eines Vorsprungs 31 des Montagerahmens 20. Hierbei kann die Wippe 30 zum Fixieren einer Batterie 130 dienen, wohingegen die Vorsprünge 31 zum Fixieren eines Kondensators/Kondensatorstapels 131 verwendet werden können.

Insbesondere kann der Montagerahmen 20 gemäß Fig. 3 genau eine Wippe 30 und genau sechs Vorsprünge 31 des Montagerahmens 40 aufweisen. Die Wippe 30 kann dabei in einem ersten Abschnitt 26 des Montagerahmens 20 angeordnet sein, der eine Begrenzung des ersten Bereiches 140 des Innenraums 14 bilden kann, der z.B. zur Aufnahme einer elektrischen Komponente in Form einer Batterie 130 dienen kann. Weiterhin können die Vorsprünge 31 des Montagerahmens 40 in einem zweiten Abschnitt 28 des Montagerahmens 20 angeordnet sein, der eine Begrenzung des dritten Bereiches 144 bilden kann, der zur Aufnahme einer elektrischen Komponente in Form eines Kondensators/Kondensatorstapels 130 dienen kann. Die Vorsprünge 31 können paarweise am zweiten Abschnitt 28 des Rahmens 20 gruppiert sein.

Vorzugsweise weist der jeweilige Vorsprung 31 des Montagerahmens 20 ein freies Ende 42 auf, wobei sich der jeweilige Vorsprung 31 zu seinem Ende 41 hin verjüngt. Weiterhin ist insbesondere vorgesehen, dass die Vorsprünge 31 jeweils in einer Aussparung 44 am zweiten Rand 24 des Montagerahmens 20 angeordnet sind bzw. in der Aussparung 44 vom Montagerahmen 20 abstehen. Hierbei erststrecken sich die Vorsprünge 31 insbesondere vor dem Fixieren der betreffenden Komponente 131 in der Montagerichtung 110. Wie weiterhin aus Fig. 3 ersichtlich ist (vgl. auch Fig. 4), weist die Wippe 30 vorzugsweise einen ersten Arm 52 und einen zweiten Arm 54 auf, die in unterschiedliche Richtungen vom Montagrahmen 20 abstehen. Bevorzugt ist der zweite Arm 54 länger als der erste Arm 52 der Wippe 30.

Wie anhand der Figuren 3 und 4 ersichtlich ist, stehen der erste und der zweite Arm 52, 54 der Wippe 30 vorzugsweise von einem Steg 70 ab, der durch eine erste Aussparung 72 des ersten Randes 22 sowie durch eine gegenüberliegende zweite Aussparung 74 des zweiten Randes 24 des Montagerahmens 20 gebildet ist. Hierbei liegt die zweite Aussparung 74 der ersten Aussparung 72 in Richtung der Montagerichtung 110 gegenüber. Entlang der Montagerichtung 110 oder auch senkrecht zum Boden 16 kann der Abstand zwischen dem ersten Rand 22 und dem Steg 70 geringer sein als der Abstand zwischen dem Steg 70 und dem zweiten Rand 24.

Die oben beschriebenen Klemmstrukturen 30, 31 können jeweils einen ersten Zustand einnehmen und sind dazu ausgebildet zum Fixieren der jeweiligen Komponente 130, 131 in einen zweiten Zustand überführt zu werden.

Fig. 3 zeigt diesbezüglich die Klemmstrukturen 30, 31 des Montagerahmens 20 im jeweiligen ersten Zustand, wobei ein Anordnen der Komponenten 130, 131 im Montagerahmen 20 möglich ist.

Im ersten Zustand erstreckt sich der jeweilige Vorsprung 31 des Montagerahmens 20 senkrecht zur ersten Ebene 60 bzw. zum Boden 16 der ersten Gerätegehäuseschale 12 auf der der Montagerahmen 20 angeordnet ist. Die Montagerichtung 110 verläuft dabei senkrecht zur ersten Ebene 60 bzw. zum Boden 16, so dass sich der jeweilige Vorsprung 31 in der Montagerichtung 110 erstreckt. Das freie Ende 42 des jeweiligen Vorsprungs 31 kann dabei senkrecht zur ersten Ebene 60 bzw. zum Boden 16 über den zweiten Rand 24 des Montagerahmens 20 hinausragen.

Wie weiterhin aus Fig. 4 ersichtlich ist, kann sich auch der zweite Arm 54 der mindestens einen Wippe 30 senkrecht zum Boden 16 (entlang der Montagerichtung 110) über den zweiten Rand 24 des Montagerahmens 20 hinaus erstrecken.

Insbesondere kann die mindestens eine Wippe 30 so konfiguriert sein, dass sich der zweite Arm 54 senkrecht zum Boden 16 bzw. zur ersten Ebene 60 erstreckt, wenn sich die Wippe im ersten Zustand befindet. Durch diese Ausrichtung der Wippe 30 (gleiches gilt für die Vorsprünge 31) kann die betreffende elektrische Komponente (hier z.B. die Batterie 130) entlang der Montagerichtung 110 in dem Montagerahmen 20 angeordnet werden.

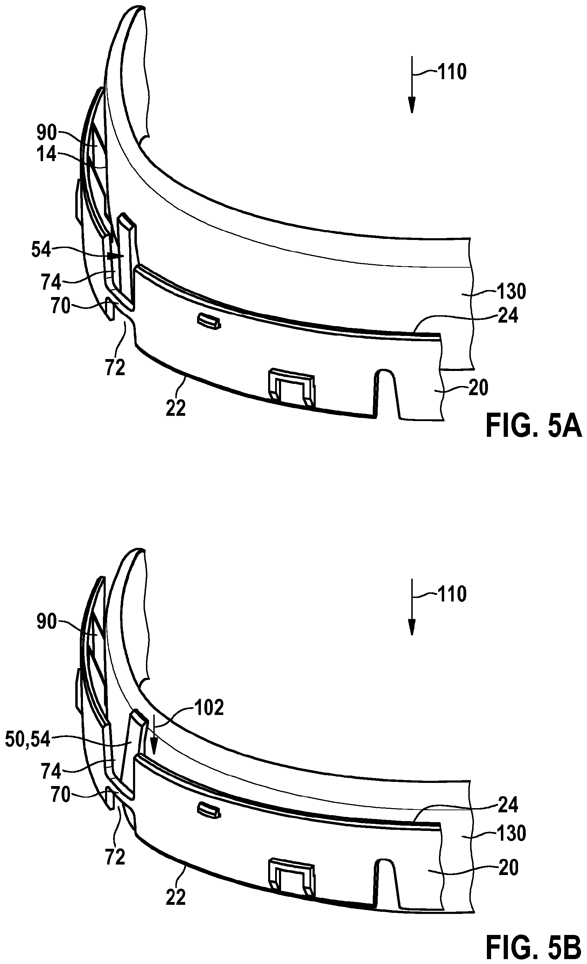

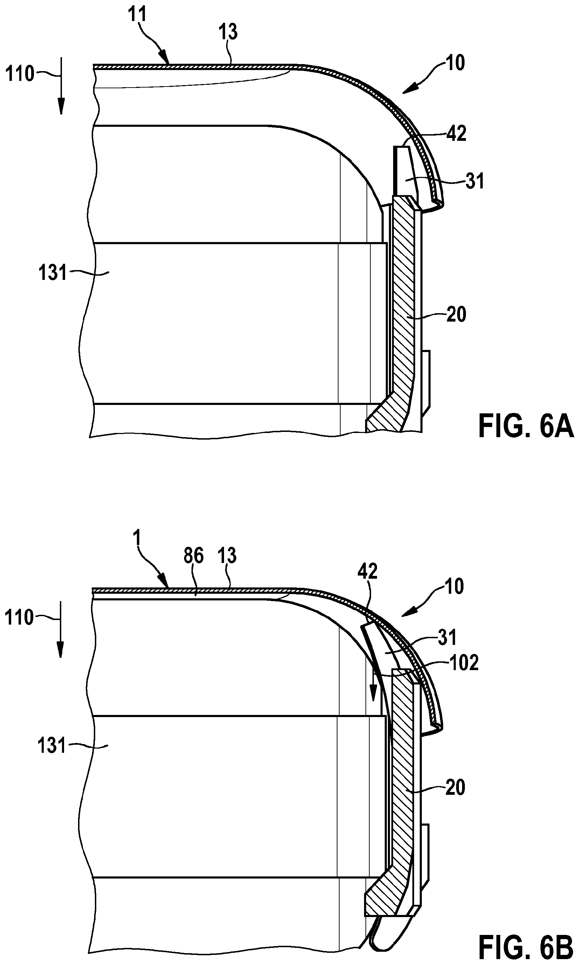

Die jeweilige Klemmstruktur 30, 31 ist erfindungsgemäß weiterhin dazu ausgebildet, von dem jeweiligen ersten Zustand in einen zweiten Zustand überführbar zu sein. Dies ist z.B. in den Figuren 4, 5A, 5B, 6A und 6B illustriert. In den Figuren 4, 5A und 5B sind der erste und der zweite Zustand der mindestens einen Wippe 30 gezeigt. Die Figuren 6A und 6B stellen den ersten und den zweiten Zustand für den Fall einer Klemmstruktur 31 in Form eines Vorsprungs 31 dar.

Fig. 4 veranschaulicht im Zusammenhang mit den Figuren 5A und 5B das Funktionsprinzip der Wippe 30. Hierzu zeigt die Fig. 4 eine Schnittansicht der Wippe 30 entlang einer Ebene senkrecht zur Erstreckungsrichtung des Steges 70. Die im ersten Zustand befindliche Wippe 30 ist grob schraffiert dargestellt, wohingegen die im zweiten Zustand befindliche Wippe 30 in feinerer Schraffur gezeigt ist.

Vorzugsweise ist der erste Arm 52 unter einem stumpfen Winkel 120 zum zweiten Arm 52 am Steg 70 angeordnet, wobei der erste Arm 52 der Wippe 30 im ersten Zustand der Wippe 30 parallel zum Boden 16 weiter in den Innenraum 14 hineinragt als der zweite Arm 54.

Hierdurch kann z. B. durch Anordnen der entsprechenden elektrischen Komponente 130 in den Montagerahmen 20 (vgl. Fig. 5A) eine Kraft 100 senkrecht zum Boden (in Richtung der Montagerichtung 110) auf den ersten Arm 52 ausgeübt werden, durch denn die Wippe 30 unter Deformation des Steges 70 vom ersten in den zweiten Zustand gekippt wird, wodurch weiterhin der zweite Arm 54 gegen die besagte Komponente 130 drückt und diese fixiert (vgl. Fig. 5B). Das Kippen der Wippe 30 kann um eine Kippachse erfolgen, die sich insbesondere entlang der Längserstreckungsrichtung bzw. Längsachse des Steges 70 erstrecken kann.

Der in den Figuren 4, 5A und 5B gezeigte Montagerahmen 20 kann zudem eine oder mehrere Rippen 90 aufweisen, mit deren Hilfe die elektrische Komponente 130 parallel zur ersten Ebene 60 bzw. parallel zum Boden 16 der ersten Gerätegehäuseschale 12 im Gerätegehäuse 10 fixiert werden kann.

Die Figuren 6A und 6B zeigen jeweils einen ausschnitthaften Querschnitt durch ein Gerätegehäuse 10 eines implantierbaren medizinischen Geräts 1 im geöffneten Zustand (Fig. 6A) und im geschlossenen Zustand (Fig. 6B) und illustrieren dabei die Funktionsweise der jeweiligen Klemmstruktur 31, wenn diese in Form eines Vorsprungs 31 des Montagerahmens 20 vorliegt. Dabei zeigt Fig. 6A einen Vorsprung 31 im ersten Zustand während Fig. 6B den Vorsprung 31 im zweiten Zustand zeigt. In beiden Fällen kann die elektrische Komponente 130 bereits in dem Montagerahmen 20 angeordnet sein, sich also im Innenraum 14 des Gerätegehäuses 10 des Implantates 1 befinden.

Im ersten Zustand (Fig. 6A) erstreckt sich der jeweilige Vorsprung 31 entlang der Montagerichtung 110. Die zweite Gerätegehäuseschale 13 kann nun in der Montagerichtung 110 zur ersten Gerätegehäuseschale 12 hin bewegt werden, um mit dieser verbunden zu werden. Dabei drückt die zweite Gerätegehäuseschale 13 gegen das Ende 42 des jeweiligen Vorsprungs 31 und deformiert diesen derart, dass der jeweilige Vorsprung gegen die elektrische Komponente drückt und diese damit senkrecht zum Boden 16 bzw. zur ersten Ebene 60 im Gerätegehäuse 10 fixiert. Dies ist in der Figur 6B gezeigt. Der jeweilige Vorsprung 31 kann dabei so deformiert werden, dass er sich an die elektrische Komponente 130 anlegen und auf die elektrische Komponente 130 eine Kraft ausübt, die eine Vektorkomponente 102 aufweist, die entlang der Montagerichtung 110 verläuft bzw. senkreckt zum Boden 16/ersten Ebene 60 orientiert ist.

## Patentansprüche

1. Implantierbares medizinisches Gerät (1), mit:
- einem Gerätegehäuse (10) aufweisend eine erste Gerätegehäuseschale (12) und eine zweite Gerätegehäuseschale (13), wobei das Gerätegehäuse (10) einen Innenraum (14) umgibt,
- einer elektrische Komponente des Gerätes (130, 131), die im Innenraum (14) angeordnet ist, wobei die elektrische Komponente (130) eine Batterie oder ein Kondensator ist,
- einem Montagerahmen (20), der im Innenraum (14) angeordnet ist und die elektrische Komponente (130, 131) umgibt,
wobei der Montagerahmen (20) mindestens eine Klemmstruktur (30, 31) aufweist, und wobei die mindestens eine Klemmstruktur (30, 31) dazu ausgebildet ist, eine Kraft (102) auf die elektrische Komponente (130) auszuüben, um die elektrische Komponente (130) im Innenraum des Gerätegehäuses (14) zu fixieren,
**dadurch gekennzeichnet, dass**
die mindestens eine Klemmstruktur (30) als eine Wippe ausgebildet ist, wobei die Wippe (30) zumindest einen ersten Arm (52) und einen zweiten Arm (54) aufweist, wobei der erste Arm (52) und der zweite Arm (54) in unterschiedliche Richtungen von einem durch den Montagerahmen (20) gebildeten Steg (70) abstehen..

2. Implantierbares medizinisches Gerät (1) nach Anspruch 1, **dadurch gekennzeichnet, dass** die mindestens eine Klemmstruktur (30, 31) dazu ausgebildet ist, mindestens einen ersten und einen zweiten Zustand einzunehmen, wobei im ersten Zustand keine Kraft durch die mindestens eine Klemmstruktur (30, 31) auf die elektrische Komponente (130, 131) ausgeübt wird, und wobei im zweitem Zustand eine Kraft (102) durch die mindestens eine Klemmstruktur (30, 31) auf die elektrische Komponente (130, 131) ausgeübt wird, um die elektrische Komponente (130, 131) im Innenraum des Gerätegehäuses (14) zu fixieren.

3. Implantierbares medizinisches Gerät (1) nach Anspruch 2, **dadurch gekennzeichnet, dass** die mindestens eine Klemmstruktur (30, 31) dazu ausgebildet ist, vom ersten Zustand in den zweiten Zustand überführt zu werden, wenn die elektrische Komponente (130) in dem Montagerahmen (20) angeordnet wird oder wenn das Gerätegehäuse (10) durch Zusammensetzen der ersten und der zweiten Gerätegehäuseschale (12, 13) geschlossen wird.

4. Implantierbares medizinisches Gerät (1) nach einem der vorhergehenden Ansprüche, **dadurch gekennzeichnet, dass** die erste Gerätegehäuseschale (12) einen Boden (16) sowie eine davon abgehende umlaufende Wandung (18) aufweist; und/oder dass die zweite Gerätegehäuseschale (13) einen Boden (16) sowie eine davon abgehende umlaufende Wandung (18) aufweist.

5. Implantierbares medizinisches Gerät (1) nach einem der Ansprüche 2 oder 3 und nach Anspruch 4, aufweisend mindestens eine weitere Klemmstruktur (31), wobei die mindestens die mindestens eine weitere Klemmstruktur (31) als ein Vorsprung des Montagerahmens (20) ausgebildet ist, wobei sich der Vorsprung des Montagerahmens (20) im ersten Zustand der mindestens einen Klemmstruktur (31) senkrecht zum Boden (16) der ersten Gerätegehäuseschale (12) oder senkrecht zum Boden (16) der zweiten Gerätegehäuseschale (13) erstreckt.

6. Implantierbares medizinisches Gerät (1) nach Anspruch 5, **dadurch gekennzeichnet, dass** der Vorsprung (31) des Montagerahmens (20) ein Ende (42) aufweist, wobei sich der Vorsprung (31) des Montagerahmens (20) zum Ende (42) des Vorsprungs (31) hin verjüngt.

7. Implantierbares medizinisches Gerät (1) nach einem der Ansprüche 5 oder 6, **dadurch gekennzeichnet, dass** zumindest ein Bereich (31, 70) der mindestens einen Klemmstruktur (30) oder der mindestens einen weiteren Klemmstruktur (31) deformierbar ist, so dass durch eine Deformation des mindestens einen Bereiches (31, 70) der mindestens einen Klemmstruktur (30) oder der mindestens einen weiteren Klemmstruktur (31) die mindestens einen Klemmstruktur (30) oder die mindestens eine weitere Klemmstruktur (31) vom ersten Zustand in den zweiten Zustand überführbar ist.

8. Implantierbares medizinisches Gerät (1) nach einem der Ansprüche 5 bis 7, **dadurch gekennzeichnet, dass** die mindestens einen Klemmstruktur (30, 31) im zweiten Zustand in Richtung des Bodens (16) der ersten Gerätegehäuseschale (12) geneigt ist und dabei gegen die elektrische Komponente (130) drückt, um die besagte Kraft (102) auf die elektrische Komponente (130) auszuüben.

9. Implantierbares medizinisches Gerät (1) nach Anspruch 1 und nach Anspruch 4, **dadurch gekennzeichnet, dass** der erste Arm (52) sich im ersten Zustand geneigt zum Boden (16) der ersten Gerätegehäuseschale (12) erstreckt und/oder dass sich der zweite Arm (54) im ersten Zustand senkrecht zum Boden (16) der ersten Gerätegehäuseschale (12) erstreckt.

10. Implantierbares medizinisches Gerät (1) nach einem der vorherigen Ansprüche, **dadurch gekennzeichnet, dass** der zweite Arm (54) länger ist als der erste Arm (52).

11. Implantierbares medizinisches Gerät (1) nach einem der vorherigen Ansprüche, **dadurch gekennzeichnet, dass** die mindestens eine im ersten Zustand befindliche Klemmstruktur (30) durch Ausüben einer Kraft (100) auf den ersten Arm (52) in den zweiten Zustand kippbar ist.

12. Implantierbares medizinisches Gerät (1) nach einem der vorherigen Ansprüche, **dadurch gekennzeichnet, dass** der Montagerahmen (20) einen ersten Rand (22) und einen gegenüberliegenden zweiten Rand (24) aufweist, wobei der Steg (70) zwischen einer Aussparung des Montagerahmens am ersten Rand (72, 22) und einer gegenüberliegenden Aussparung des Montagerahmens am zweiten Rand (74, 24) verläuft.

13. Implantierbares medizinisches Gerät (1) nach einem der vorhergehenden Ansprüche, **dadurch gekennzeichnet, dass** der Montagerahmen (20) eine weitere Klemmstruktur (30, 31) aufweist, wobei die weitere Klemmstruktur (30, 31) als ein weiterer Vorsprung (31) des Montagerahmens (20) ausgebildet ist, oder wobei die weitere Klemmstruktur als eine weitere Wippe ausgebildet ist, wobei insbesondere die weitere Wippe zumindest einen ersten und einen zweiten Arm aufweist, die insbesondere in unterschiedlichen Richtungen von einem durch den Montagerahmen gebildeten weiteren Steg abstehen.

## Claims

1. An implantable medical device (1), having:
- a device housing (10), comprising a first device housing shell (12) and a second device housing shell (13), wherein the device housing (10) surrounds an interior (14),
- an electrical component (130, 131) arranged in the interior (14), the electrical component (130) being a battery or a capacitor,
- a mounting frame (20), which is arranged in the interior (14) and surrounds the electrical component (130, 131),
- wherein the mounting frame (20) comprises at least one clamping structure (30, 31), and wherein the at least one clamping structure (30, 31) is designed to exert a force (102) onto the electrical component (130) in order to fix the electrical component (130) in the interior of the device housing (14), and
wherein the electrical component (130) is a battery or a capacitor,
**characterized in that**
the at least one clamping structure (30) is designed as a rocker, wherein the rocker (30) comprises at least a first arm (52) and a second arm (54), wherein the first arm (52) and the second arm (54) protrude in different directions from a web (70) formed by the mounting frame (20).

2. The implantable medical device (1) according to claim 1, **characterised in** the at least one clamping structure (30, 31) is designed to assume at least a first and a second state, wherein in the first state no force is exerted by the at least one clamping structure (30, 31) onto the electrical component (130, 131), and wherein in the second state a force (102) is exerted by the at least one clamping structure (30, 31) onto the electrical component (130, 131) in order to fix the electrical component (130, 131) in the interior of the device housing (14).

3. The implantable medical device (1) according to claim 2, **characterised in that** the at least one clamping structure (30, 31) is designed to be transferred from the first state into the second state when the electrical component (130) is arranged in the mounting frame (20) or when the device housing (10) is closed by assembling together the first and the second device housing shell (12, 13).

4. The implantable medical device (1) according to any one of the preceding claims, **characterised in that** the first device housing shell (12) comprises a base (16) and a circumferential wall (18) starting therefrom; and/or **in that** the second device housing shell (13) comprises a base (16) and a circumferential wall (18) starting therefrom.

5. The implantable medical device (1) according to either one of claims 2 or 3 and according to claim 4, **characterised in that** the at least one clamping structure (31) is designed as a protrusion of the mounting frame (20), wherein the protrusion of the mounting frame (20) in the first state of the at least one clamping structure (31) extends perpendicularly to the base (16) of the first device housing shell (12) or perpendicularly to the base (16) of the second device housing shell (13).

6. The implantable medical device (1) according to claim 5, **characterised in that** the protrusion (31) of the mounting frame (20) has an end (42), wherein the protrusion (31) of the mounting frame (20) tapers towards the end (42) of the protrusion (31).

7. The implantable medical device (1) according to either one of claims 5 or 6, **characterised in that** at least one region (31, 70) of the at least one clamping structure (30, 31) is deformable, such that by way of a deformation of the at least one region (31, 70) of the at least one clamping structure (30), the at least one clamping structure (30, 31) can be transferred from the first state into the second state.

8. The implantable medical device (1) according to any one of claims 5 to 7, **characterised in that** the at least one clamping structure (30, 31) in the second state is inclined in the direction of the base (16) of the first device housing shell (12) and thus presses against the electrical component (130) in order to exert said force (102) onto the electrical component (130).

9. The implantable medical device (1) according to claim 1 and according to claim 4, **characterised in that** the first arm (52) in the first state extends inclined towards the base (16) of the first device housing shell (12), and/or **in that** the second arm (54) extends in the first state perpendicularly to the base (16) of the first device housing shell (12).

10. The implantable medical device (1) according to either one of claims 9 or 10, **characterised in that** the second arm (54) is longer than the first arm (52).

11. The implantable medical device (1) according to any one of claims 9 to 11, **characterised in that** the at least one clamping structure (30) in the first state can be tilted into the second state by exerting a force (100) onto the first arm (52).

12. The implantable medical device (1) according to any one of claims 9 to 12, **characterised in that** the mounting frame (20) has a first edge (22) and an opposite second edge (24), wherein a bar (70) runs between a recess of the mounting frame on the first edge (72, 22) and an opposite recess of the mounting frame on the second edge (74, 24).

13. The implantable medical device (1) according to any one of the preceding claims, **characterised in that** the mounting frame (20) has a further clamping structure (30, 31), wherein the further clamping structure (30, 31) is designed as a further protrusion (31) of the mounting frame (20), or wherein the further clamping structure is designed as a further rocker, wherein in particular the further rocker comprises at least a first and a second arm, which in particular protrude in different directions from a further web formed by the mounting frame.

## Revendications

1. Appareil médical implantable (1) pourvu :
- d'un boitier d'appareil (10) présentant une première coque de boitier d'appareil (12) et une deuxième coque de boitier d'appareil (13), où le boitier d'appareil (10) entoure un espace intérieur (14),
- d'un composant électrique de l'appareil (130, 131) qui est disposé dans l'espace intérieur (14), où le composant électrique (130) est une pile ou un condensateur,
- d'un cadre de montage (20) qui est disposé dans l'espace intérieur (14) et qui entoure le composant électrique (130, 131),
dans lequel le cadre de montage (20) présente au moins une structure de serrage (30, 31) et dans lequel l'au moins une structure de serrage (30, 31) est conçue pour exercer une force (102) sur le composant électrique (130) afin de fixer le composant électrique (130) dans l'espace intérieur du boitier d'appareil (14),
**caractérisé en ce que**
l'au moins une structure de serrage (30) est conçue sous forme d'une bascule, où la bascule (30) présente au moins un premier bras (52) et un deuxième bras (54), où le premier bras (52) et le deuxième bras (54) ressortent dans diverses directions d'une tige (70) formée par le cadre de montage (20).

2. Appareil médical implantable (1) selon la revendication 1, **caractérisé en ce que** l'au moins une structure de serrage (30, 31) est conçue pour adopter au moins un premier et un deuxième état, où, aucune force n'est exercée sur le composant électrique (130, 131) par l'au moins une structure de serrage (30, 31) dans le premier état, et où une force (102) est exercée sur le composant électrique (130, 131) par l'au moins une structure de serrage (30, 31) dans le deuxième état, afin de fixer le composant électrique (130, 131) dans l'espace intérieur du boitier d'appareil (14).

3. Appareil médical implantable (1) selon la revendication 2, **caractérisé en ce que** l'au moins une structure de serrage (30, 31) est conçue pour être transférée du premier état vers le deuxième état lorsque le composant électrique ((130) est disposé dans le cadre de montage (20) ou lorsque le boitier d'appareil (10) est fermé par l'assemblage des première et deuxième coques de boitier d'appareil (12, 13).

4. Appareil médical implantable (1) selon l'une des revendications précédentes, **caractérisé en ce que** la première coque de boitier d'appareil (12) présente un fond (16) ainsi qu'une paroi périphérique (18) partant de celui-ci ; et/ou que la deuxième coque de boitier d'appareil (13) présente un fond (16) ainsi qu'une paroi périphérique (18) partant de celui-ci.

5. Appareil médical implantable (1) selon l'une des revendications 2 ou 3 et selon la revendication 4, présentant au moins une nouvelle structure de serrage (31), où l'au moins une nouvelle structure de serrage (31) est conçue sous forme d'une protubérance du cadre de montage (20), où la protubérance du cadre de montage (20) s'étend dans le premier état de l'au moins une structure de serrage (31) de manière verticale par rapport au fond (16) de la première coque de boitier d'appareil (12) ou de manière verticale par rapport au fond (16) de la deuxième coque de boitier d'appareil (13).

6. Appareil médical implantable (1) selon la revendication 5, **caractérisé en ce que** la protubérance (31) du cadre de montage (20) présente une extrémité (42), où la protubérance (31) du cadre de montage (20) s'amenuise vers l'extrémité (42) de la protubérance (31).

7. Appareil médical implantable (1) selon l'une des revendications 5 ou 6, **caractérisé en ce qu'**au moins une région (31, 70) de l'au moins une structure de serrage (30), ou de l'au moins une nouvelle structure de serrage (31), est déformable de sorte que, par une déformation de l'au moins une région (31, 70) de l'au moins une structure de serrage (30) ou de l'au moins une nouvelle structure de serrage (31), l'au moins une structure de serrage (30), ou l'au moins une nouvelle structure de serrage (31), peut être transférée du premier état vers le deuxième état.

8. Appareil médical implantable (1) selon l'une des revendications 5 à 7, **caractérisé en ce que** l'au moins une structure de serrage (30, 31) est inclinée en direction du fond (16) de la première coque de boitier d'appareil (12) dans le deuxième état et se presse ainsi contre le composant électrique (130) afin d'exercer ladite force (102) sur le composant électrique (130).

9. Appareil médical implantable (1) selon la revendication 1 et selon la revendication 4, **caractérisé en ce que** le premier bras (52) s'incline dans le premier état vers le fond (16) de la première coque de boitier d'appareil (12), et/ou que le deuxième bras (54) dans le premier état s'étend perpendiculairement par rapport au fond (16) de la première coque de boitier d'appareil (12).

10. Appareil médical implantable (1) selon l'une des revendications précédentes, **caractérisé en ce que** le deuxième bras (54) est plus long que le premier bras (52).

11. Appareil médical implantable (1) selon l'une des revendications précédentes, **caractérisé en ce que** l'au moins une structure de serrage (30) se trouvant dans le premier état peut être basculée vers le deuxième état par l'exercice d'une force (100) sur le premier bras (52).

12. Appareil médical implantable (1) selon l'une des revendications précédentes, **caractérisé en ce que** le cadre de montage (20) présente un premier bord (22) et un deuxième bord (24) situé à l'opposé, où la tige (70) s'étend entre un évidement du cadre de montage sur le premier bord (72, 22) et un évidement situé à l'opposé du cadre de montage sur le deuxième bord (74, 24).

13. Appareil médical implantable (1) selon l'une des revendications précédentes, **caractérisé en ce que** le cadre de montage (20) présente une nouvelle structure de serrage (30, 31), où la nouvelle structure de serrage (30, 31) est conçue comme une nouvelle protubérance (31) du cadre de montage (20) ou, où la nouvelle structure de serrage est conçue comme une nouvelle bascule, où, en particulier, la nouvelle bascule présente au moins un premier et un deuxième bras qui ressortent dans diverses directions à partir de la nouvelle tige formée par le cadre de montage.
